# EUROPÄISCHE PATENTSCHRIFT

(11) **EP 1 514 546 B1**
(45) Veröffentlichungstag und Bekanntmachung des Hinweises auf die Patenterteilung: **23.05.2007**
(21) Anmeldenummer: 04028508.2
(22) Anmeldetag: 09.11.2001
(51) Int. Cl.: A61K 31/46, A61K 31/137, A61P 11/00, A61P 11/06

(54) **Arzneimittelkompositionen auf der Basis von Tiotropiumsalzen und Salzen des Salmeterols**
Medicament compositions based on tiotropium salts and on salmeterol salts
Compositions de médicaments a bàse de sels de tiotropium et de sels de salmeterol

(30) Priorität: 13.11.2000 DE 10056104
(43) Veröffentlichungstag der Anmeldung: 16.03.2005
(62) Teilanmeldung aus: 01989446.8
(73) Patentinhaber: Boehringer Ingelheim Pharma GmbH & Co.KG, 55216 Ingelheim am Rhein (DE)
(72) Erfinder: Nagel, Juergen, Dr., deceased (DE); Nagel, Hilke, 55543 Bad Kreuznach (DE); Schmelzer, Christel, Dr., 55218 Ingelheim (DE)

(56) Entgegenhaltungen:
- WO-A-00/69468
- DE-A- 19 847 970
- BARNES P J: "Chronic obstructive pulmonary disease: new opportunities for drug development" TRENDS IN PHARMACOLOGICAL SCIENCES, ELSEVIER TRENDS JOURNAL, CAMBRIDGE, GB, Bd. 19, Nr. 10, Oktober 1998 (1998-10), Seiten 415-423, XP004156947 ISSN: 0165-6147
- REES P J: "BRONCHODILATORS IN THE THERAPY OF CHRONIC OBSTRUCTIVE PULMONARY DISEASE" EUROPEAN RESPIRATORY MONOGRAPH, EUROPEAN RESPIRATORY SOCIETY JOURNALS LTD., SHEFFIELD,, GB, Bd. 7, Nr. 7, 1998, Seiten 135-149, XP000937584 ISSN: 1025-448X

## Beschreibung

Die vorliegende Erfindung betrifft neurtige Arzneimittelkompositionen auf der Basis von Tiotropiumsalzen und Salzen des Salmeterols, Verfahren zu deren Herstellung sowie deren Verwendung bei der Therapie von Atemwegserkrankungen.

### Hintergrund der Erfindung

Die Verbindung Tiotropiumbromid, ein Salz des Tiotropiums, ist aus der Europäischen Patentanmeldung EP 418 716 A1 bekannt und weist die folgende chemische Struktur auf:

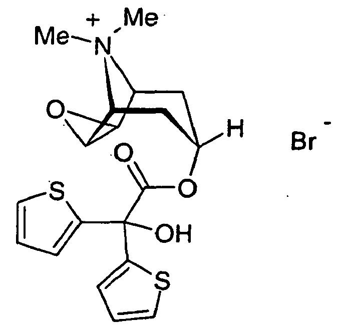

Diese Verbindung kann auch auch durch den chemischen Namen (1α,2β,4β,5α,7β)-7-[(hydroxydi-2-thienylacetyl)oxy]-9,9-dimethyl-3-oxa-9-azoniatricyclo[3.3.1.0^{2,4}] nonane-bromid bezeichnet werden und besitzt wertvolle pharmakologische Eigenschaften. Die Bezeichnung Tiotropium ist im Rahmen der vorliegenden Erfindung als Bezugnahme auf das freie Kation zu verstehen.

Sie, wie auch andere Salze des Tiotropiums, stellt ein hochwirksames Anticholiriergikum dar und kann deshalb bei der Therapie von Asthma oder COPD (chronic obstructive pulmonary disease = chronisch obstruktive Lungenerkrankung) einen therapeutischen Nutzen entfalten.

Die Applikation von Tiotropiumsalzen erfolgt vorzugsweise auf inhalativem Wege. Hierbei können geeignete Inhaltionspulver, die in geeignete Kapseln (Inhaletten) abgefüllt mittels entsprechender Pulverinhalatoren appliziert werden, zum Einsatz kommen. Alternativ dazu kann eine inhalative Anwendung auch durch Applikation geeigneter Inhalationsaerosole erfolgen. Hierzu zählen auch pulverförmige Inhalationsaerosole, die beispielsweise HFA134a, HFA227 oder deren Gemisch als Treibgas enthalten. Die inhalative Applikation kann ferner mittels geeigneter Lösungen des Tiotropiumsalzes erfolgen.

### Detaillierte Beschreibung der Erfindung

Überraschenderweise wurde gefunden, daß ein unerwartet vorteilhafter therapeutischer Effekt, insbesondere ein synergistischer Effekt bei der Behandlung von entzündlichen oder obstruktiven Atemwegserkrankungen beobachtet wird, wenn ein oder mehrere Tiotropiumsalze (**1**) in Kombination mit ein oder mehreren Salmeterolsalzen (**2**) zur Anwendung gelangen.

Hierdurch lassen sich beispielsweise unerwünschte Nebenwirkungen, die häufig bei der Applikation von β-Mimetika, wie Salmeterol, am Menschen beobachtet werden deutlich verringern. Als zentrale Nebenwirkungen von β-Mimetika seien beispielsweise allgemeine Unruhe, Erregung, Schlaflosigkeit, Angst, Fingerzittern, Schweißausbrüche und Kopfschmerzen genannt.

Die Bezeichnung Tiotropium ist im Rahmen der vorliegenden Erfindung als Bezugnahme auf das freie Kation (**1'**) zu verstehen. Eine Bezugnahme auf Salmeterol ist im Rahmen der vorliegenden Erfindung als Bezugnahme auf die freie Base (**2'**) zu verstehen.

Die erfindungsgemäßen Wirkstoffkombinationen sind überraschenderweise ferner sowohl durch einen raschen Wirkungseintritt, als auch durch eine langandauernde Wirkdauer gekennzeichnet. Dies ist von hoher Bedeutung für das Wohlbefinden des Patienten, da er einerseits nach Applikation der Kombination eine rasche Verbesserung seines Zustands verspürt und andererseits aufgrund der langen Wirkdauer eine einmal pro Tag erfolgende Applikation ausreichend ist.

Ein Aspekt der vorliegenden Erfindung betrifft ein Arzneimittel, welches ein oder mehrere Tiotropiumsalze (**1**) und ein oder mehrere Salmeterolsalze (**2**), gegebenfalls in Form ihrer Solvate oder Hydrate enthält, dadurch gekennzeichnet, dass es sich um ein Inhalationspulver handelt, welches **1** und **2** im Gemisch mit geeignetem physiologisch unbedenklichem Hilfsstoff enthält, der eine mittlere Teilchengröße zwischen 10 und 150µm aufweist.

Die vorliegende Erfindung betrifft ferner die Verwendung des vorstehend genannten Inhalationspulvers zur Herstellung eines therapeutisch wirksame Mengen von **1** und **2** enthaltenden Arzneimittels zur Behandlung von entzündlichen oder obstruktiven Atemwegserkrankungen, insbesondere von Asthma oder COPD.

Unter den im Rahmen der vorliegenden Erfindung einsetzbaren Tiotropiumsalzen **1** sind die Verbindungen zu verstehen, die neben Tiotropium als Gegenion (Anion) Chlorid, Bromid, Iodid, Methansulfonat, para-Toluolsulfonat oder Methylsulfat enthalten. Im Rahmen der vorliegenden Erfindung sind von allen Tiotropiumsalzen das Methansulfonat, Chlorid, Bromid oder Iodid bevorzugt, wobei dem Methansulfonat oder dem Bromid besondere Bedeutung zukommt. Von erfindungsgemäß herausragender Bedeutung ist das Tiotropiumbromid.

Unter Salzen des Salmeterols **2** werden erfindungsgemäß pharmazeutisch verträgliche Salze verstanden, die ausgewählt aus den Salzen der Salzsäure, Bromwasserstoffsäure, Schwefelsäure, Phosphorsäure, Methansulfonsäure, Essigsäure, Fumarsäure, Bernsteinsäure, Milchsäure, Zitronensäure, Weinsäure, Xinafonsäure oder Maleinsäure sind. Gegebenenfalls können zur Herstellung der Salmeterolsalze auch Mischungen der vorgenannten Säuren eingesetzt werden. Erfindungsgemäß bevorzugt sind die Salmeterolsalze **2** ausgewählt aus der Gruppe bestehend aus Hydrochlorid, Hydrobromid, Sulfat, Phosphat und Methansulfonat. Besonders bevorzugt sind die Salze von **2** ausgewählt aus Hydrochlorid und Sulfat, von denen die Sulfate besonders bevorzugt sind. Erfindungsgemäß von herausragender Bedeutung ist Salmeterol x ½ H₂SO₄.

In den erfindungsgemäßen Wirkstoffkombinationen aus **1** und **2** können die Bestandteile **1** und **2** in Form ihrer Enantiomere, Gemische der Enantiomere oder in Form der Racemate enthalten sein.
Die Verhältnisse, in denen die beiden Wirkstoffe **1** und **2** in die erfindungsgemäßen Wirkstoffkombinationen eingesetzt werden können, sind variabel. Die Wirkstoffe **1** und **2** können gegebenfalls in Form ihrer Solvate oder Hydrate vorliegen. Je nach Wahl der Salze **1** bzw. **2** variieren die im Rahmen der vorliegenden Erfindung einsetzbaren Gewichtsverhältnisse aufgrund des unterschiedlichen Molekulargewichts der verschiedenen Salzformen. Den nachfolgend angegebenen Gewichtsverhältnissen wurden daher das Tiotropiumkation **1'** sowie die freie Base des Salmeterols **2'** zugrunde gelegt. Die erfindungsgemäßen Wirkstoffkombinationen können **1'** und **2'** in Gewichtsverhältnissen enthalten, die in einem Bereich von 1:300 bis 30:1, bevorzugt von 1:230 bis 20:1, besonders bevorzugt von 1:150 bis 10:1, ferner bevorzugt von 1:50 bis 5:1, besonders bevorzugt von 1:35 bis 2:1. Erfindungsgemäß von besonderem Interesse sind Arzneimittel, enthaltend die Kombination aus **1'** und **2'** in einem Gewichtsverhältnis im Bereich von 1:25 bis 1:1. bevorzugt in einem Bereich von 1:10 bis 1:2, besonders bevorzugt in einem Bereich von 1:5 bis 1:2,5.

Beispielsweise und ohne den Umfang der Erfindung darauf zu beschränken, können bevorzugte erfindungsgemäße Kombinationen aus **1** und **2** Tiotropium **1'** und Salmeterol **2'** in den folgenden Gewichtsverhältnissen enthalten: 1:40; 1:20; 1:11,1; 1:10; 1:5,6; 1:5; 1:2,8; 1:2,5; 1:1,4; 1:1,25; 1,44:1, 1,6:1.

Die Anwendung der erfindungsgemäßen Arzneimittel enthaltend die Kombinationen aus **1** und **2** erfolgt üblicherweise so, daß Tiotropium **1'** und Salmeterol **2'** gemeinsam in Dosierungen von 0,01 bis 10000µg, bevorzugt von 0,1 bis 2000µg, besonders bevorzugt von 1 bis 1000µg, ferner bevorzugt von 5 bis 500µg, erfindungsgemäß bevorzugt von 10 bis 200µg, bevorzugt von 20 bis 100µg, höchst bevorzugt von 30 bis 70µg pro Einmalgabe.
Beispielsweise enthalten erfindungsgemäße Kombinationen aus **1** und **2** eine solche Menge an Tiotropium **1'** und Salmeterol **2',** daß die Gesamtdosierung pro Einmalgabe 30µg, 35µg, 45µg, 55µg, 60µg, 65µg, 90µg, 105µg, 110µg, 110µg, 140µg oder ähnliches beträgt. Bei diesen Dosierungsbereichen sind die Wirkstoffe **1'** und **2'** in den vorhergehend beschriebenen Gewichtsverhältnissen enthalten.

Beispielsweise und ohne den Umfang der Erfindung darauf zu beschränken, können die erfindungsgemäßen Kombinationen aus **1** und **2** eine solche Menge an Tiotropium **1'** und Salmeterol **2'** enthalten, daß pro Einmalgabe 5µg **1'** und 25µg **2',** 5µg **1'** und 50µg **2**', 5µg **1'** und 100µg **2'**, 5µg **1'** und 200µg **2'**, 10µg **1'** und 25µg **2'**, 10µg **1'** und 50µg **2'**, 10µg **1'** und 100µg **2'**, 10µg **1'** und 200µg **2'**, 18µg **1'** und 25µg **2'**, 18µg **1'** und 50µg **2'**, 18µg **1'** und 100µg **2'**, 18µg **1'** und 200µg **2'**, 20µg **1'** und 25µg **2'**, 20µg **1'** und 50µg **2'**, 20µg **1'** und 100µg **2'**, 20µg **1'** und 200µg **2'**, 36µg **1'** und 25µg **2'**, 36µg **1'** und 50µg **2'**, 36µg **1'** und 100µg **2'**, 36µg **1'** und 200µg **2'**, 40µg **1'** und 25µg **2'**, 40µg **1'** und 50µg **2'**, 40µg **1'** und 100µg **2'** oder 40µg **1'** und 200µg **2'** appliziert werden.

Wird als erfindungsgemäß bevorzugte Kombination aus **1** und **2** die Wirkstoffkombination verwendet, in der **1** Tiotropiumbromid bedeutet und in der **2** für Salmeterol x ½H₂SO₄ steht, entsprechen die vorstehend beispielhaft genannten pro Einmalgabe applizierten Wirkstoffmengen von **1'** und **2'** den nachfolgenden pro Einmalgabe applizierten Mengen an **1** und **2**: 6µg **1** und 27,9µg **2**, 6µg **1** und 55,9µg **2,** 6µg **1** und 111,8µg **2**, 6µg **1** und 223,6µg **2,** 12µg **1** und 27,9µg **2,** 12µg **1** und 55,9µg **2,** 12µg **1** und 111,8µg **2,** 12µg **1** und 223,6µg **2**, 21,7µg **1** und 27,9µg **2**, 21,7µg **1** und 55,9µg **2,** 21,7µg **1** und 111,8µg **2,** 21,7µg **1** und 223,6µg **2,** 24,1µg **1** und 27,9µg **2,** 24,1µg **1** und 55,9µg **2,** 24,1µg **1** und 111,8µg **2,** 24,1µg **1** und 223,6µg **2,** 43,3µg **1** und 27,9µg **2,** 43,3µg **1** und 55,9µg **2,** 43,3µg **1** und 111,8µg **2,** 43,3µg **1** und 223,6µg **2,** 48,1 µg **1** und 27,9µg **2**, 48,1 µg **1** und 55,9µg **2,** 48,1 µg **1** und 111,8µg **2** oder 48,1µg **1** und 223,6µg **2**.

Wird in der erfindungsgemäß bevorzugten Kombination aus **1** und **2**, in der **2** für Salmeterol x ½H₂SO₄ steht als **1** beispielsweise das Tiotropiumbromidmonohydrat eingesetzt, entsprechen die vorstehend beispielhaft genannten pro Einmalgabe applizierten Wirkstoffmengen von **1'** und **2'** den nachfolgenden pro Einmalgabe applizierten Mengen an **1** und **2:** 6,2µg **1** und 27,9µg **2,** 6,2µg **1** und 55,9µg **2,** 6,2µg 1 und 111,8µg 2, 6,2µg **1** und 223,6µg **2,** 12,5µg **1** und 27,9µg **2,** 12,5µg **1** und 55,9µg **2**, 12,5µg **1** und 111,8µg **2,** 12,5µg **1** und 223,6µg **2,** 22,5µg **1** und 27,9µg **2,** 22,5µg **1** und 55,9µg **2,** 22,5µg **1** und 111,8µg **2,** 22,5µg **1** und 223,6µg **2,** 25µg **1** und 27,9µg **2,** 25µg **1** und 55,9µg **2,** 25µg **1** und 111,8µg **2,** 25µg **1** und 223,6µg **2,** 45µg **1** und 27,9µg **2,** 45µg **1** und 55,9µg **2,** 45µg **1** und 111,8µg **2**, 45µg **1** und 223,6µg **2,** 50µg **1** und 27,9µg **2,** 50µg **1** und 55,9µg **2,** 50µg **1** und 111,8µg **2** oder 50µg **1** und 223,6µg **2**.

Die Applikation der erfindungsgemäßen Wirkstoffkombinationen aus **1** und **2** erfolgt bevorzugt auf inhalativem Wege. Hierzu müssen die Bestandteile **1** und **2** in inhalierbaren Darreichungsformen bereitgestellt werden.
Als inhalierbare Darreichungsformen kommen erfindungsgemäß Inhalationspulver in Betracht. Erfindungsgemäße Inhalationspulver enthaltend die Wirkstoffkombination aus **1** und **2** können aus einem Gemisch der genannten Wirkstoffe mit physiologisch verträglichen Hilfsstoffen bestehen. Die im Rahmen der vorliegenden Erfindung einsetzbaren Darreichungsformen werden im nachfolgenden Teil der Beschreibung detailliert beschrieben.

### Inhalationspulver enthaltend die erfindungsgemäßen Wirkstoffkombinationen aus 1 und 2:

Die erfindungsgemäßen Inhaltionspulver können **1** und **2** im Gemisch mit geeigneten physiologisch unbedenkliche Hilfsstoffen-enthalten, die eine mittlere Teilchengröße zwischen 10 und 150µm aufweisen.

Zur Darstellung der erfindungsgemäßen Inhalationspulver können die folgenden physiologisch unbedenklichen Hilfsstoffe zur Anwendung gelangen: Monosaccharide (z.B. Glucose oder Arabinose), Disaccharide (z.B. Lactose, Saccharose, Maltose), Oligo- und Polysaccharide (z.B. Dextrane), Polyalkohole (z.B. Sorbit, Mannit, Xylit), Salze (z.B. Natriumchlorid, Calciumcarbonat) oder Mischungen dieser Hilfsstoffe miteinander. Bevorzugt gelangen Mono- oder Disaccharide zur Anwendung, wobei die Verwendung von Lactose oder Glucose, insbesondere, aber nicht ausschließlich in Form ihrer Hydrate, bevorzugt ist. Als besonders bevorzugt im Sinne der Erfindung gelangt Lactose, höchst bevorzugt Lactosemonohydrat als Hilfsstoff zur Anwendung.

Die Hilfsstoffe weisen im Rahmen der erfindungsgemäßen Inhaltionspulver eine mittlere Teilchengröße zwischen 10 und 150µm, besonders bevorzugt zwischen 15 und 80µm auf. Gegebenenfalls kann es sinnvoll erscheinen, den vorstehend genannten Hilfststoffen feinere Hilfsstofffraktionen mit einer mittleren Teilchengröße von 1 bis 9µm beizumischen. Letztgenannte feinere Hilfsstoffe sind ebenfalls ausgewählt aus der vorstehend genannten Gruppe an einsetzbaren Hilfsstoffen. Schließlich wird zur Herstellung der erfindungsgemäßen Inhaltionspulver mikronisierter Wirkstoff **1** und **2**, vorzugsweise mit einer mittleren Teilchengröße von 0,5 bis 10µm, besonders bevorzugt von 1 bis 6µm, der Hilfsstoffmischung beigemischt. Verfahren zur Herstellung der erfindungsgemäßen Inhaltionspulver durch Mahlen und Mikronisieren sowie durch abschließendes Mischen der Bestandteile sind aus dem Stand der Technik bekannt.

Die erfindungsgemäßen Inhalationspulver können mittels aus dem Stand der Technik bekannten Inhalatoren appliziert werden.
Erfindungsgemäße Inhalationspulver, die neben **1** und **2** ferner einen physiologisch unbedenklichen Hilfsstoff enthalten, können beispielsweise mittels Inhalatoren appliziert werden, die eine einzelne Dosis aus einem Vorrat mittels einer Meßkammer, wie er in der US 4570630A beschrieben wird, oder über andere apparative Vorrichtungen, wie sie in der DE 36 25 685 A beschrieben werden, dosieren. Vorzugsweise werden die erfindungsgemäßen Inhalationspulver, die neben **1** und **2** physiologisch unbedenkliche Hilfsstoff enthalten, allerdings in Kapseln abgefüllt (zu sogenannten Inhaletten), die in Inhalatoren wie beispielsweise in der WO 94/28958 beschrieben, zur Anwendung gelangen.
Sollen die erfindungsgemäßen Inhalationspulver im Sinne der vorstehend gennannten bevorzugten Anwendung in Kapseln (Inhaletten) abgefüllt werden, bieten sich Füllmengen von 1 bis 30mg, bevorzugt von 3 bis 20mg, bevorzugt 5 bis 10 mg Inhalationspulver pro Kapsel an. Diese enthalten erfindungsgemäß entweder gemeinsam oder jeweils die bereits vorstehend für **1'** und **2'** genannten Dosierungen pro Einmalgabe.

Die folgenden Beispiele dienen einer weitergehenden Erläuterung der vorliegenden Erfindung.

### Ausgangsmaterialien

### Tiotropiumbromid:

Das in den nachfolgenden Formulierungsbeispielen eingesetzte Tiotropiumbromid kann wie in der Europäischen Patentanmeldung EP 418 716 A1 beschrieben, erhalten werden.

Zur Herstellung der erfindungsgemäßen Inhalationspulver kann ebenfalls kristallines Tiotropiumbromidmonohydrat eingesetzt werden. Dieses kristalline Tiotropiumbromidmonohydrat ist gemäß nachfolgend beschriebener Vorgehensweise erhältlich.
In einem geeigneten Reaktionsgefäß werden in 25,7 kg Wasser 15,0 kg Tiotropiumbromid eingetragen. Die Mischung wird auf 80-90°C erhitzt und bei gleichbleibender Temperatur solange gerührt, bis eine klare Lösung entsteht. Aktivkohle (0,8 kg), wasserfeucht, wird in 4,4 kg Wasser aufgeschlämmt, diese Mischung in die Tiotropiumbromid-haltige Lösung eingetragen und mit 4,3 kg Wasser nachgespült. Die so erhaltene Mischung wird wenigstens 15 min bei 80-90°C gerührt und anschließend über einen beheizten Filter in einen auf 70°C Manteltemperatur vorgewärmten Apparat filtriert. Der Filter wird mit 8,6 kg Wasser nachgespült. Der Apparateinhalt wird mit 3-5°C pro 20 Minuten auf eine Temperatur von 20-25°C abgekühlt. Mit Kaltwasserkühlung wird der Apparat auf 10-15°C weiter abgekühlt und die Kristallisation durch mindestens einstündiges Nachrühren vervollständigt. Das Kristallisat wird über einen Nutschentrockner isoliert, der isolierte Kristallbrei mit 9 L kaltem Wasser (10-15°C) und kaltem Aceton (10-15°C) gewaschen. Die erhaltenen Kristalle werden bei 25°C über 2 Stunden im Stickstoffstrom getrocknet.
Ausbeute : 13,4 kg Tiotropiumbromidmonohydrat (86 % d. Th.)

Das so erhaltene kristalline Tiotropiumbromidmonohydrat wird nach bekannten Verfahren mikronisiert, um den Wirkstoff in Form der mittleren Teilchengröße bereitzustellen, die den erfindungsgemäßen Spezifikationen entspricht.

### Salmeterol x ½H₂SO₄:

Wird in den nachfolgenden Ausführungsbeispielen auf Salmeterol x ½H₂SO₄ Bezug genommen, so wurde dieses wie folgt erhalten:
Eine Suspension aus 2.5 g (4.15 mmol) Salmeterolxinafoat wird in 6 ml Ethanol gelöst. Unter Rühren wird eine Lösung von 0.14 ml 98%iger Schwefelsäure langsam zu der Suspension gegeben. Es wird bis zur vollständigen Lösung auf 35-40 °C erwärmt. Anschließend wird mit 10ml Diethylether verdünnt und die Lösung mit Salmeterolsulfat angeimpft. Das Salmeterolsulfat wird nach 1.5 Stunden abgesaugt und mit je 20 ml kaltem Ethanol, Aceton und Diethylether gewaschen.
Man erhält 1.5 g (78%) Salmeterol-½-sulfat

### Formulierungsbeispiele

### Inhalationspulver:

1)

| **Bestandteile** | **µg pro Kapsel** |
|---|---|
| Tiotropiumbromid | 10,8 |
| Salmeterol x ½ H₂SO₄ | 27,9 |
| Lactose | 4961,3 |
| **Summe** | 5000 |

2)

| **Bestandteile** | **µg pro Kapsel** |
|---|---|
| Tiotropiumbromid | 21,7 |
| Salmeterol x ½ H₂SO₄ | 55,9 |
| Lactose | 4922,4 |
| **Summe** | 5000 |

3)

| **Bestandteile** | **µg pro Kapsel** |
|---|---|
| Tiotropiumbromid x H₂O | 22,5 |
| Salmeterol x ½H₂SO₄ | 55,9 |
| Lactose | 4921,6 |
| **Summe** | 5000 |

## Patentansprüche

1. Arzneimittel **gekennzeichnet durch** einen Gehalt an einem oder mehreren Tiotropiumsalzen (**1**) in Kombination mit einem oder mehreren Salmeterolsalzen (**2**), gegebenenfalls in Form ihrer Enantiomere, Gemische der Enantiomere oder in Form der Racemate, gegebenenfalls in Form der Solvate oder Hydrate , **dadurch gekennzeichnet, dass** es sich um ein Inhalationspulver handelt, welches **1** und **2** im Gemisch mit geeignetem physiologisch unbedenklichem Hilfsstoff enthält, der eine mittlere Teilchengröße zwischen 10 und 150µm aufweist.

2. Arzneimittel nach Anspruch 1, **dadurch gekennzeichnet, daß** **1** in Form des Chlorids, Bromids, Iodids, Methansulfonats, para-Toluolsulfonats oder Methylsulfats, bevorzugt in Form des Bromids enthalten ist.

3. Arzneimittel nach einem der Ansprüche 1 oder 2, **dadurch gekennzeichnet, daß** **2** ausgewählt ist aus den Salzen der Salzsäure, Bromwasserstoffsäure, Schwefelsäure, Phosphorsäure, Methansulfonsäure, Essigsäure, Fumarsäure, Bernsteinsäure, Milchsäure, Zitronensäure, Xinafonsäure oder Maleinsäure.

4. Arzneimittel nach Anspruch 3, **dadurch gekennzeichnet, daß** **2** ausgewählt ist aus den Salzen Hydrochlorid, Hydrobromid, Sulfat, Phosphat und Methansulfonat, bevorzugt aus Hydrochlorid und Sulfat.

5. Arzneimittel nach einem der Ansprüche 1 bis 4, **dadurch gekennzeichnet, daß** die Gewichtsverhältnisse von Tiotropium **1'** zu Salmeterol **2'**,in einem Bereich von 1:300 bis 30:1, bevorzugt von 1:230 bis 20:1 liegen.

6. Arzneimittel nach einem der Ansprüche 1 bis 5, **dadurch gekennzeichnet, daß** eine einmaliger Applikation einer Dosierung der Wirkstoffkombination **1'** und **2'** von 0,01 bis 1000µg, bevorzugt von 0,1 bis 200µg entspricht.

7. Arzneimittel nach einem der Ansprüche 1 bis 6, **dadurch gekennzeichnet, daß** es ein Inhalationspulver ist, welches **1** und **2** im Gemisch mit geeigneten physiologisch unbedenkliche Hilfsstoffen ausgewählt aus der Gruppe bestehend aus Monosaccharide, Disaccharide, Oligo- und Polysaccharide, Polyalkohole, Salze, oder Mischungen dieser Hilfsstoffe miteinander enthält.

8. Kapseln **gekennzeichnet durch** einen Gehalt an Inhalationspulver nach einem der Ansprüche 1 bis 7.

9. Verwendung einer Zusammensetzung nach einem der Ansprüche 1 bis 8 zur Herstellung eines Medikaments zur Behandlung von Atemwegserkrankungen.

10. Verwendung nach Anspruch 9 zur Herstellung eines Medikaments zur Behandlung von Asthma oder COPD.

## Claims

1. Pharmaceutical composition, **characterised in that** it contains one or more tiotropium salts (**1**) combined with one or more salmeterol salts (**2**), optionally in the form of the enantiomers, mixtures of enantiomers or in the form of the racemates thereof, optionally in the form of the solvates or hydrates, **characterised in that** it is an inhalable powder which contains **1** and **2** in admixture with a suitable physiologically acceptable excipient which has an average particle size of between 10 and 150 µm.

2. Pharmaceutical composition according to claim 1, **characterised in that 1** is contained in the form of the chloride, bromide, iodide, methanesulphonate, *para*-toluenesulphonate or methylsulphate, preferably in the form of the bromide.

3. Pharmaceutical composition according to one of claims 1 or 2, **characterised in that** **2** is selected from the salts of hydrochloric acid, hydrobromic acid, sulphuric acid, phosphoric acid, methanesulphonic acid, acetic acid, fumaric acid, succinic acid, lactic acid, citric acid, xinafonic acid or maleic acid.

4. Pharmaceutical composition according to claim 3, **characterised in that 2** is selected from the hydrochloride, hydrobromide, sulphate, phosphate and methanesulphonate salts, preferably from hydrochloride and sulphate.

5. Pharmaceutical composition according to one of claims 1 to 4, **characterised in that** the ratios by weight of tiotropium **1'** to salmeterol **2'** are in a range from 1:300 to 30:1, preferably from 1:230 to 20:1.

6. Pharmaceutical composition according to one of claims 1 to 5, **characterised in that** a single administration corresponds to a dosage of the active substance combination **1'** and **2'** of from 0.01 to 1000 µg, preferably from 0.1 to 200 µg.

7. Pharmaceutical composition according to one of claims 1 to 6, **characterised in that** it is an inhalable powder which contains **1** and **2** in admixture with suitable physiologically acceptable excipients selected from among monosaccharides, disaccharides, oligo- and polysaccharides, polyalcohols, salts, or mixtures of these excipients with one another.

8. Capsules, **characterised in that** they contain inhalable powders according to one of claims 1 to 7.

9. Use of a composition according to one of claims 1 to 8 for preparing a medicament for the treatment of respiratory diseases.

10. Use according to claim 9 for preparing a medicament for the treatment of asthma or COPD.

## Revendications

1. Médicament **caractérisé par** une teneur en un ou plusieurs sels de tiotropium (**1**) en combinaison avec un ou plusieurs sels de salmétérol (**2**), éventuellement sous forme de leurs énantiomères, de mélanges des énantiomères ou sous forme des racémates, éventuellement sous forme des solvates ou hydrates, **caractérisé en ce qu'**il s'agit d'une poudre pour inhalation qui contient **1** et **2** en mélange avec un adjuvant physiologiquement acceptable approprié, qui présente une taille particulaire moyenne entre 10 et 150 µm.

2. Médicament selon la revendication 1 **caractérisé en ce que 1** est contenu sous forme du chlorure, bromure, iodure, méthanesulfonate, paratoluènesulfonate ou méthylsulfate, de préférence sous forme du bromure.

3. Médicament selon l'une des revendications 1 ou 2 **caractérisé en ce que** **2** est choisi parmi les sels de l'acide chlorhydrique, de l'acide bromhydrique, de l'acide sulfurique, de l'acide phosphorique, de l'acide méthanesulfonique, de l'acide acétique, de l'acide fumarique, de l'acide succinique, de l'acide lactique, de l'acide citrique, de l'acide xinafoïque ou de l'acide maléique.

4. Médicament selon la revendication 3 **caractérisé en ce que** **2** est choisi parmi les sels chlorhydrate, bromhydrate, sulfate, phosphate et méthanesulfonate, de préférence parmi chlorhydrate et sulfate.

5. Médicament selon l'une des revendications 1 à 4 **caractérisé en ce que** les rapports massiques du tiotropium **1'** au salmétérol **2'** sont situés dans une plage de 1 : 300 à 30 : 1, de préférence de 1 : 230 à 20 : 1.

6. Médicament selon l'une des revendications 1 à 5 **caractérisé en ce qu'**une application unique correspond à une posologie de la combinaison de principes actifs **1'** et **2'** de 0,01 à 1000 µg, de préférence de 0,1 à 200 µg.

7. Médicament selon l'une des revendications 1 à 6 **caractérisé en ce que** c'est une poudre pour inhalation qui contient **1** et **2** en mélange avec des adjuvants physiologiquement acceptables appropriés choisis dans le groupe consistant en les monosaccharides, les disaccharides, les oligo- et polysaccharides, les polyalcools, les sels ou les mélanges de ces adjuvants entre eux.

8. Capsules **caractérisées par** une teneur en poudre pour inhalation selon l'une des revendications 1 à 7.

9. Utilisation d'une composition selon l'une des revendications 1 à 8 pour la production d'un médicament pour le traitement des maladies des voies respiratoires.

10. Utilisation selon la revendication 9 pour la production d'un médicament pour le traitement de l'asthme ou de la COPD.
